# EUROPEAN PATENT SPECIFICATION

(11) **EP 1 299 424 B1**
(45) Date of publication and mention of the grant of the patent: **03.09.2008**
(21) Application number: 01961446.0
(22) Date of filing: 03.07.2001
(51) Int. Cl.: C08B 37/00, A61K 31/716, A61P 3/08, A61P 17/02, A61P 7/00, A61P 9/00, A61P 1/10, A61P 37/04, A23L 1/10, A23L 1/052

(54) **COLD WATER SOLUBLE $g(b)-GLUCAN PRODUCT AND PROCESS FOR PREPARING THE SAME**
IN KALTEM WASSER LÖSLICHES -G(B)-GLUKANPRODUKT UND VERFAHREN ZU DESSEN HERSTELLUNG
PRODUIT DERIVE DE $g(b)-GLUCANE SOLUBLE DANS L'EAU FROIDE ET SON PROCEDE DE FABRICATION

(30) Priority: 03.07.2000 NZ 50554500
(43) Date of publication of application: 09.04.2003
(73) Proprietor: Granate Seed Limited, Wellington (NZ); Roxdale Foods Limited, Auckland (NZ)
(72) Inventor: MORGAN, Keith, Raymond, Petone (NZ)
(74) Representative: O'Connell, Maura
(86) International application number: PCT/NZ2001/000133
(87) International publication number: WO 2002/002645

(56) References cited:
- WO-A-00/56268
- US-A- 5 512 287
- TAPPY L ET AL: "EFFECTS OF BREAKFAST CEREALS CONTAINING VARIOUS AMOUNTS OF BETA-GLUCAN FIBERS ON PLASMA GLUCOSE AND INSULIN RESPONSES IN NIDDMSUBJECTS" DIABETES CARE, AMERICAN DIABETES ASSOCIATION, ALEXANDRIA, VA, US, vol. 19, no. 8, August 1996 (1996-08), pages 831-834, XP001027914 ISSN: 0149-5992
- IZAWA ET AL.: 'Relationship between structure and solubility of (1->3),(1->4)-beta-D-glucan from Barley' JOURNAL OF THE AMERICAN SOCIETY OF BREWING CHEMISTS vol. 51, 1993, pages 123 - 127, XP002964483
- CHOCT M.: 'Feed non-starch polysaccharides: Chemical structures and nutritional significance' FEED MILLING INTERNATIONAL June 1997, pages 13 - 26, XP002964484
- MORGAN ET AL CARBOHADRATE RESEARCH vol. 315, 1999, pages 169 - 179

## Description

### FIELD OF INVENTION

This invention relates to a novel β-glucan product and a process for preparing it. In particular, the invention relates to a β-glucan product which is readily soluble in cold water.

### BACKGROUND

The term "β-glucan" refers to those polysaccharides which comprise D-glucopyranosyl units which are linked together by (1→3) and (1→4) β-linkages. β-Glucans occur naturally in many cereal grains such as oats and barley. The molecular weight of β-glucan molecules occurring in cereals is typically 200 to 2000 kiloDaltons.

β-Glucan is desirable as a food additive, for example, to impart texture ("mouth feel") to foods. β-Glucan is also useful for preparing edible films for food coatings. β-Glucan may also be used to add bulk to foods and has the advantage of having a neutral flavour.

β-Glucan is also desirable as a therapeutic agent. There is evidence that β-glucan can lower serum cholesterol levels, heal wounds, moderate glycaemic response, and alleviate constipation. β-Glucan can actively bind to specific cell receptors and therefore may be useful for the treatment of a wide variety of disorders or diseases.

The known methods for extracting β-glucan from cereal grains, such as oats and barley, involve several steps. Firstly, the cereal grain is milled to a flour prior to extracting β-glucan from the flour using warm or hot water or an aqueous alkali solution. The milling step facilitates release of the β-glucan from the cereal. The aqueous extract of β-glucan is then separated from the solid flour residue. Finally, the β-glucan is recovered from the extract.

The known methods of recovering the β-glucan from the aqueous extract include precipitation of the β-glucan using a water miscible solvent, such as alcohol, or by freezing and then thawing the extract to give a precipitate of β-glucan which can be recovered by filtration or centrifugation. Also, the β-glucan solution can be concentrated and treated to form a gel, and then dried.

The β-glucan products obtained, once dried to a solid or formed into a gel, can be redissolved in water. However, for β-glucan with high average molecular weight (> 200,000 Daltons) solubility in cold water is relatively low and it is therefore desirable that the water is heated to a temperature above approximately 70 °C so that the β-glucan will dissolve. This makes it difficult to incorporate the β-glucan into a processed food or a pharmaceutical formulation.

It is also known that β-glucan with high average molecular weight tends to form a viscous solution in water, whereas β-glucan with low average molecular weight (< 200,000 Daltons) tends to form a gel when dissolved in water.

It has now been found that recovering low average molecular weight β-glucan from an aqueous solution before the solution can form a gel gives a β-glucan solid which is readily soluble in cold water.

It is therefore an object of this invention to provide a novel β-glucan product which is soluble in cold water and which sets to form a gel, or to provide a useful alternative product. It is also an object of the invention to provide a process for preparing the β-glucan product, or at least to provide a useful alternative process.

### SUMMARY OF INVENTION

In one aspect of the invention there is provided β-glucan solid which is greater than 2% by weight soluble in water when mixed with water at a temperature below approximately 50 °C and which β-glucan is capable of forming a gel.

Preferably the β-glucan is soluble in water at a temperature below approximately 30 °C. It is also preferred that the β-glucan is capable of forming a gel below approximately 70 °C.

Preferably the β-glucan is greater than 50% by weight soluble in water when mixed with water at a temperature below approximately 50 °C, more preferably greater than 80% by weight, and even more preferably approximately 100% by weight.

In a preferred embodiment of the invention the β-glucan has an average molecular weight of less than approximately 200,000 Daltons. It is also preferred that the β-glucan forms a soft gel when dissolved in water at a concentration above approximately 2% or a fluid gel when dissolved in water at a concentration below approximately 2%.

Preferably the β-glucan is prepared from β-glucan which has been extracted from a cereal such as barley, oats or wheat.

In a second aspect of the invention there is provided a process for preparing β-glucan of the first aspect of this invention from an aqueous solution of β-glucan by removing water from the aqueous solution before any gelation begins to occur to give β-glucan solid, or by adding a water-miscible organic solvent, such as ethanol, to the aqueous solution before any gelation begins to occur to cause precipitation of β-glucan solid.

In one embodiment of this aspect of the invention the aqueous solution of β-glucan is prepared by dissolving β-glucan in water at a temperature greater than approximately 70 °C, preferably greater than approximately 90 °C or even greater than approximately 100 °C. In an alternative embodiment the aqueous solution of β-glucan is prepared by heating an aqueous β-glucan gel, optionally with additional water, at a temperature greater than approximately 70 °C, preferably greater than approximately 90 °C or even greater than approximately 100 °C.

Preferably the water is removed from the aqueous solution by evaporating the water at a temperature higher than the melting temperature of a gel capable of being formed by the β-glucan.

In a preferred embodiment of the second aspect of the invention the aqueous solution of β-glucan is obtained by milling cereal grain to form a flour, mixing the flour with water to form a slurry of an aqueous solution of β-glucan and a solid residue, and separating the aqueous solution from the solid residue. Optionally, the aqueous solution is purified before carrying out the steps of the process of the second aspect of the invention.

The invention also provides a pharmaceutical composition containing β-glucan of this invention.

The invention further provides a method for lowering serum cholesterol levels in an animal comprising administering to animal β-glucan of this invention.

The invention also provides a method for healing a wound in an animal comprising administering to the animal β-glucan of this invention.

The invention also provides a method of regulating glycaemic response in an animal comprising administering to the animal β-glucan of this invention.

The invention also provides a method for stimulating the immune system in an animal comprising administering to the animal β-glucan of this invention.

The invention also provides a method of alleviating constipation in an animal comprising administering to the animal β-glucan of this invention.

The invention further provides a use of β-glucan of this invention as a food ingredient. Preferably the β-glucan is used as an ingredient in a functional food or a nutraceutical. The invention additionally provides a processed food containing β-glucan of this invention.

Furthermore, the invention provides the use of β-glucan in the preparation of an edible film. The invention additionally provides an edible film containing β-glucan of this invention.

### DETAILED DESCRIPTION

For the purposes of this invention, the term "cold-water soluble" means soluble in water at a temperature which is below the temperature at which the β-glucan dissolved in water would form a gel. β-Glucan aqueous gels typically form at temperatures below about 60°C. Generally, the cold water soluble β-glucan products of this invention are soluble in water at a temperature above 0°C and form aqueous solutions containing up to approximately 10% β-glucan.

A soft gel is a gel which flows under deformation. A fluid gel is a free flowing solution containing gel particles.

It has been found that recovering β-glucan from an aqueous solution before the solution begins to form a gel gives a β-glucan solid which is readily soluble in cold water. The aqueous solution of β-glucan may be prepared by aqueous extraction of the β-glucan from cereal. Alternatively, the solution may be prepared by dissolving solid β-glucan in hot water. β-glucan solid is typically only sparingly soluble in cold water and dissolution is usually effected only at temperatures of 70°C or more. Another method of preparing the aqueous solution of β-glucan is to heat an aqueous β-glucan gel, optionally with additional water.

Whether or not β-glucan forms a gel when dissolved in water depends principally on the average molecular weight of the β-glucan. Low molecular weight β-glucan (below approximately 200,000 Daltons) usually forms a gel whereas high molecular weight β-glucan is less likely to form a gel when dissolved in water and is more likely to simply form a viscous solution. For β-glucan which is cold-water soluble and which has a sufficiently low average molecular weight, a gel can be readily formed by dissolving in cold water.

The β-glucan product of this invention can be dissolved in cold water to form a soft gel or a fluid gel. Usually a soft gel will form where the concentration of β-glucan in the gel is above approximately 2% and a fluid gel will form if the concentration of β-glucan in the gel is less than about 2%. The time taken for gelation to occur depends on the average molecular weight of the β-glucan and the concentration of the β-glucan in water. A 5% β-glucan solution may form a gel within 15 minutes whereas a 1 % β-glucan solution may take a day or longer to form a gel.

The β-glucan of this invention is especially advantageous for the incorporation of β-glucan into pharmaceuticals, foods, nutraceuticals and any other substance where it is desired to incorporate β-glucan. The process of incorporating β-glucan into these substances can be greatly simplified if either no or minimal heating is required.

The β-glucan of this invention is useful for a range of therapies. These include the lowering of serum cholesterol levels, wound healing, the regulation of glycaemic response, the stimulation of the immune system, and the alleviation of constipation. It is thought that β-glucan can actively bind to specific cell receptors and is therefore useful for treating a variety of diseases or disorders.

The physical properties of the β-glucan of this invention make it desirable as a food ingredient. The effects of its incorporation into a processed food include altering texture and increasing bulk. In particular, the β-glucan of the invention is useful in functional foods and nutraceuticals. Functional foods and nutraceuticals are those foods which have specific health benefits.

Film coatings are desirable in many food applications. The β-glucan of this invention can be used to prepare such films which have the advantage of being edible.

The invention is now described with reference to the following examples but is not to be construed as limited thereto.

### EXAMPLES

### Example 1

A β-glucan solid was produced in a pilot plant production run which involved the following steps:
1. *Pearling and sieving*. Pearled barley was milled in a hammer mill and the flour produced was sieved on a 150 µm nylon mesh. The material that passed through the sieve was further sieved on a 90 µm nylon mesh. The flour fractions that were retained on the 150 µm and 90 µm mesh were used in the extraction of the β-glucan.
2. *Extraction.* The flour fractions (202 kg) were mixed with water (1400 L) to which had been added a cellulase (1g, Sigma EC 3.2.1.4 from *Penicillium funiculosum*) and a xylanase (40 ml, Shearzyme, Novo Nordisk). The extraction was continued for 60 min at a temperature of 53 °C.
3. *Separation of solids.* The flour/water mixture was fed through a Sharples decanter at a feed rate of 1500 L/h. Fines in the supernatant from the Sharples decanter were removed on an Alfa centrifuge producing 950 L of β-glucan extract.
4. *Separation of protein.* The protein in the extract was precipitated by heating the extract at 90 °C for 30 min. The precipitated protein was then removed on an Alfa centrifuge which produced 850 L of extract.
5. *Concentration.* The extract was concentrated on a Luwa wiped evaporator to a volume of 40 L.
6. *Gelation.* On cooling to room temperature the extract formed a gel. The gel was then cooled to 5 °C to strengthen the gel.
7. *Washing.* The gel was washed with 400 L of water and the water removed on the Alfa centrifuge. The washings were repeated twice more.
8. *Drying.* The gel was sprayed dried to yield. 500 g of β-glucan solid.

### Example 2

a) A sample of β-glucan solid (0.32 g) prepared in Example 1 was mixed with water (1.6 ml) in a stainless steel pressure vessel and the vessel sealed. The vessel was heated at 140 °C for 12 min in an aluminium block heater, which allowed for efficient thermal transfer. The vessel was then cooled to room temperature and the aqueous solution of β-glucan was removed and mixed with additional water (3.2 ml). The mixture was then poured into 50 ml of rapidly stirred absolute ethanol. The fibrous precipitate that formed was filtered and oven dried at 110 °C.
b) As for a) except the vessel was heated at 130 °C for 12 min.
c) As for a) except the vessel was heated at 100 °C for 15 min.

For each sample of a), b) and c), the fibrous precipitate (0.15 g) was vigorously mixed with water (10 ml) at room temperature (approximately 20 °C). This caused most of the precipitate to dissolve forming a moderately viscous solution. The viscosity of each solution was measured. For a), the viscosity was found to be about 28 mPas but this increased to 31 mPas after 1 hour. For b), the viscosity was found to be 24 mPas but this increased to 38 mPas after 1 hour. For c), the viscosity remained at 64 mPas.

### Example 3

a) A sample of the β-glucan solid, (0.16 g) prepared in Example 1 was mixed with water (1.6 ml) in a stainless steel pressure vessel and the vessel sealed. The vessel was heated at 120 °C for 15 min in a block heater, which allowed for efficient thermal transfer. The vessel was then cooled to room temperature. Gelation started occurring after about 3 hours.
b) As for a) except the vessel was heated at 100 °C for 20 min. On cooling the vessel it was found that gelation occurred after about 45 min.
c) As for a) except the vessel was heated at 90 °C for 20 min. On cooling the vessel it was found that gelation occurred after about 10 min.

### Example 4

a) A sample of the β-glucan solid (0.32 g) prepared in Example 1 was mixed with water (1.6 ml) in a stainless steel pressure vessel and the vessel sealed. The vessel was heated at 90 °C for 15 min in an aluminium block heater, which allowed for efficient thermal transfer. The vessel was then cooled to room temperature and the aqueous solution of β-glucan was removed and mixed with additional water (3.2 ml). The mixture was then poured into 50 ml of rapidly stirred absolute ethanol. The fibrous precipitate that formed was filtered and oven dried at 110 °C. Some of the dried fibrous precipitate (0.15 g) was shaken vigorously with water (3 ml) to form a β-glucan solution. After about 1.5 h the solution had set to a soft gel.
b) As for a) except the vessel was heated at 100 °C for 15 min. Some of the dried fibrous precipitate (0.15 g) was shaken vigorously with water (3 ml) to form a β-glucan solution. After about 1.5 h the solution had set to a soft gel.
c) As for a) except the vessel was heated at 80 °C for 15 min. Some of the dried fibrous precipitate (0.15 g) was shaken vigorously with water (3 ml) but only partly dissolved. After about 1.5 h a soft gel had formed.
d) As for a) except that vessel was heated at 130 °C for 15 min and the mixture was poured into a 50/50 mixture of water/ethanol (50 ml). This formed a precipitate of small fibres, which dissolved very readily in water.

### Example 5

a) A low molecular weight β-glucan was used in this experiment. It was prepared by aqueous extraction of flour from a hulled variety of barley (solids to liquid ratio was 1:5) at a temperature of 45 °C for 7 hours. After removal of the solids by centrifugation, the supernatant was frozen for 12 hours then thawed. The precipitate in the thawed solution was filtered and dried. It was purified by redissolving in water at a temperature of about 90 °C, filtering the hot solution, and repeating the freeze/thaw and drying steps. The average molecular weight, as determined by a combination of size exclusion chromatography and laser light scattering, was found to be about 12,000 Daltons.
   A sample of the dried β-glucan (0.32 g) was mixed with water (1.6 ml) in a stainless steel pressure vessel and the vessel sealed. The vessel was heated at 130 °C for 12 min in an aluminium block heater, which allowed for efficient thermal transfer. The vessel was then cooled to room temperature and the aqueous solution of β-glucan was removed and mixed with additional water (3.2 ml). The mixture was then poured into 50 ml of rapidly stirred absolute ethanol. The precipitate that formed, consisting of small fibres, was filtered and oven dried at 110 °C. Some of the dried fibrous precipitate (0.15 g) was shaken vigorously with water (3 ml) to dissolve the precipitate. After about 0.5 h a soft gel had formed.
b) Preparation was as for a) except the extraction was for only 2 hours. The molecular weight, as determined by a combination of size exclusion chromatography and laser light scattering, was found to be about 55,000 Daltons.
   A sample of the dried β-glucan (0.32 g) was mixed with water (1.6 ml) in a stainless steel pressure vessel and the vessel sealed. The vessel was heated at 130 °C for 12 min in an aluminium block heater, which allowed for efficient thermal transfer. The vessel was then cooled to room temperature and the aqueous solution of β-glucan was removed and mixed with additional water (3.2 ml). The mixture was then poured into 50 ml of rapidly stirred absolute ethanol. The precipitate that formed, consisting of small fibres, was filtered and oven dried at 110 °C. Some of the dried fibrous precipitate (0.15 g) was shaken vigorously with water (3 ml) to dissolve the precipitate. After about 1 hour a soft gel has formed.

### Example 6

A gelling β-glucan product of about 80 % purity (prepared as in Example 1) was redissolved in water at 95 °C to form a 2 % by weight solution and the solution was then frozen. The frozen solution was thawed leaving a fibrous precipitate which was filtered, washed well with water, then ethanol, and dried at 70 °C to give a β-glucan solid. The viscosity of a 1 % solution of this material indicated that it had an average molecular weight of 45,000 Daltons. The purified material was determined to be 94 % β-glucan.

### Example 7

a) Purified β-Glucan (prepared as in Example 6) was dissolved in water at 95 °C forming a β-glucan solution (10 % by weight). The solution was immediately poured onto a stainless-steel surface which was at a temperature of about 150 °C. After most of the water had evaporated the β-glucan formed a film which peeled off the hot surface. The film was further dried in an oven at 70 °C.
b) Purified β-Glucan (prepared as in Example 6) was dissolved in water at 95 °C forming a β-glucan solution (10 % by weight). The solution was heated in a bomb at 150 °C for 15 min which had good thermal contact with the heater. The bomb was cooled and the solution was poured onto a stainless-steel surface which was at a temperature of about 150 °C. After most of the water had evaporated the β-glucan formed a film which peeled off the hot surface. The film was further dried in an oven at 70 °C.

### Example 8

The solubility of cold-water soluble β-glucan was compared to that of a gelling β-glucan prepared by the freeze-thaw method in Example 6. Three solutions were prepared:
a) was formed by vigourously shaking the product from part a) of Example 7 for 5 min with water (1 % β-glucan by weight) at room temperature.
b) was formed by vigourously shaking the product from part b) of Example 7 for 5 min with water (1 % β-glucan by weight) at room temperature.
c) was formed by vigourously shaking the β-glucan solid formed by the freeze/thaw method of Example 6 for 5 min with water (1 % β-glucan by weight) at room temperature.

The solutions prepared above were filtered through Whatmans No. 1 filter paper under vacuum. For a), a small amount of viscous material remained on the filter paper, whereas for b) there was no residue on the filter paper. For c), although there was swelling of the β-glucan on contact with the water, it appeared that most the β-glucan remained undissolved and was removed by filtration. For each solution the β-glucan content of the filtered solutions was determined. Results are reported in Table 1 where the β-glucan content is given as the % amount that dissolved.

**Table 1**

| ***Solution*** | ***Dissolved* β*-glucan %*** |
|---|---|
| a | 88 |
| b | 97 |
| c | 2 |

Results indicate that β-glucan prepared by Examples 7 a) and 7 b) are mostly cold-water soluble, whereas the product prepared by the freeze/thaw method of Example 6 is only slightly soluble.

### Example 9

The change in viscosity of cold-water soluble β-glucan with time under continuous shear rate of 50 s⁻¹ was determined and compared to that of a material prepared by the freeze/thaw process. The viscosity was determined on Haake visocmeter fitted with a NV viscosity cell.

Two solutions were prepared:
a) was formed by occasionally shaking the β-glucan solid formed by the freeze/thaw method of Example 6 with water (5 % β-glucan by weight) at 95 °C for 10 min.
b) was formed by vigourously shaking for 5 min the product from part b) of Example 7 for 5 min with water (5 % β-glucan by weight) until most of the product had dissolved.

For a), there was an initial rise in viscosity from about 50 mPas to about 100 mPas over a time period of about 1 h. After 2.5 h another rise in viscosity over a period of 1.5 h to about 500 mPas was observed. After this the solution showed signs of shear thinning and the viscosity decreased to 300 mPas.

For b), the viscosity was initially 50 mPas and after 4 h increased over a period of 1.5 h to 400 mPascal and then shear thinned to a value of 150 mPascals.

These results suggest that the gelling properties of the cold water soluble material prepared in a bomb at 150 °C may be slightly different to the that prepared by the freeze-thaw method.

### Example 10

The change in viscosity of cold water soluble β-glucan with time under a regime of intermittent shear (continuous shear at a rate of 50 s⁻¹ for 60 s and no shear for 2000 s) was determined and compared to that of a material prepared by the freeze/thaw process. The viscosity was determined on Haake visocmeter fitted with a NV viscosity cell.

Two solutions were prepared:
a) was formed by occasionally shaking the β-glucan solid formed by the freeze/thaw method of Example 6 with water (3 % β-glucan by weight) at 95 °C for 10 min.
b) was formed by vigourously shaking for 5 min the product from part a) of Example 7 for 5 min with water (3 % β-glucan by weight) until most of the product had dissolved and then the solution was heated to 95 °C for 10 min.

Results for both a) and b) showed a steady increase in viscosity to values of 600 to 750 mPas. For a) the increase occurred over a time period of 10 h, whereas for b) the period was 12 h.

These results indicate that the the cold-water soluble material prepared by dissolution of the β-glucan solid at 95 °C has similar properties to that of the material prepared by the freeze/thaw process.

### INDUSTRIAL APPLICABILITY

The β-glucan of this invention has a number of uses because if its solubility in water at low temperature and because of its ability to form a gel. The uses include therapeutic uses such as lowering serum cholesterol levels, wound healing, regulating glycaemic response, stimulating the immune system, and the alleviation of constipation. Other uses include texture altering or bulking ingredient in foods, nutraceutical or functional food ingredients, and the preparation of edible films.

## Claims

1. β-Glucan solid which is greater than 2% by weight soluble in water when mixed with water at a temperature below approximately 50 °C and which β-glucan solid is capable of forming a gel.

2. β-Glucan as claimed in claim 1 wherein the temperature is below approximately 30 °C.

3. β-glucan as claimed in claim 1 or claim 2 which is capable of forming a gel below approximately 70 °C.

4. β-glucan as claimed in any one of claims 1 to 3 which is greater than 50% by weight, preferably greater than 80% by weight, more preferably approximately 100% by weight, soluble in water when mixed with water at a temperature below approximately 50 °C.

5. β-Glucan as claimed in any one of claims 1 to 4 which has an average molecular weight of less than approximately 200,000 Daltons.

6. β-Glucan as claimed in any one of claims 1 to 5 which is capable of forming a soft gel when dissolved in water at a concentration above approximately 2% by weight, or a fluid gel when dissolved in water at a concentration below approximately 2% by weight.

7. β-Glucan as claimed in any one of claims 1 to 6 which has been obtained from a cereal, the cereal being preferably selected from barley, oats or wheat.

8. A process for preparing β-glucan as claimed in claim 1 from an aqueous solution of β-glucan including:
- removing water from the aqueous solution before any gelation begins to occur to give β-glucan solid, or
- adding a water-miscible organic solvent to the aqueous solution before any gelation begins to occur to cause precipitation of β-glucan solid.

9. A process as claimed in claim 8 wherein the aqueous solution of β-glucan is prepared by dissolving β-glucan in water at a temperature greater than approximately 70 °C, preferably greater than approximately 90 °C, more preferably greater than approximately 100 °C .

10. A process as claimed in claim 8 wherein the aqueous solution of β-glucan is prepared by heating an aqueous β-glucan gel, optionally with additional water, at a temperature greater than approximately 70 °C, preferably greater than approximately 90 °C, more preferably greater than approximately 100 °C.

11. A process as claimed in any one of claims 8 to 10 wherein the water is removed from the aqueous solution by evaporation, the water being preferably evaporated at a temperature higher than the melting temperature of a gel capable of being formed by the β-glucan.

12. A process as claimed in any one of claims 8 to 11 wherein the water-miscible organic solvent is ethanol.

13. A process as claimed in any one of claims 8 to 12 wherein the aqueous solution of β-glucan is obtained by:
- milling cereal grain to form a flour,
- mixing the flour with water to form a slurry of an aqueous solution of β-glucan and a solid residue, and
- separating the aqueous solution from the solid residue.

14. A process as claimed in claim 13 wherein the aqueous solution is purified before carrying out the steps of claim 8.

15. A pharmaceutical composition containing β-glucan as claimed in any one of claims 1 to 7 and a pharmaceutically acceptable carrier.

16. A use of β-glucan as claimed in claim 1 as a food ingredient, the food ingredient being preferably an ingredient in a functional food or a nutraceutical.

17. A use of β-glucan as claimed in claim 1 in the preparation of an edible film.

18. A food, preferably a functional food, containing β-glucan as claimed in claim 1.

19. A nutraceutical containing β-glucan as claimed in claim 1.

20. An edible film containing β-glucan as claimed in claim 1.

## Patentansprüche

1. β-Glucan-Feststoff, der zu mehr als 2 Gewichts-% in Wasser löslich ist, wenn er mit Wasser bei einer Temperatur unterhalb von ungefähr 50 °C gemischt wird, und welcher β-Glucan-Feststoff geeignet ist, ein Gel zu bilden.

2. β-Glucan nach Anspruch 1, worin die Temperatur unterhalb von ungefähr 30 °C liegt.

3. β-Glucan nach Anspruch 1 oder Anspruch 2, das geeignet ist, unterhalb von ungefähr 70 °C ein Gel zu bilden.

4. β-Glucan nach einem der Ansprüche 1 bis 3, das zu mehr als 50 Gewichts-%, bevorzugt zu mehr als 80 Gewichts-%, bevorzugter zu ungefähr 100 Gewichts-% in Wasser löslich ist, wenn es mit Wasser bei einer Temperatur unterhalb von ungefähr 50 °C gemischt wird.

5. β-Glucan nach einem der Ansprüche 1 bis 4, das ein durchschnittliches Molekulargewicht von geringer als ungefähr 200 000 Dalton hat.

6. β-Glucan nach einem der Ansprüche 1 bis 5, das geeignet ist, ein Weichgel zu bilden, wenn es in Wasser bei einer Konzentration über ungefähr 2 Gewichts-% gelöst wird, oder ein Flüssiggel zu bilden, wenn es in Wasser bei einer Konzentration unterhalb von ungefähr 2 Gewichts-% gelöst wird.

7. β-Glucan nach einem der Ansprüche 1 bis 6, das aus einem Getreide erhalten wurde, wobei das Getreide bevorzugt aus Gerste, Hafer oder Weizen ausgewählt wurde.

8. Verfahren zur Herstellung von β-Glucan nach Anspruch 1 aus einer wässrigen Lösung von β-Glucan, das Folgendes einschließt:
- Entfernen von Wasser aus der wässrigen Lösung, bevor jedwede Gelierung aufzutreten beginnt, um den β-Glucan-Feststoff zu liefern, oder
- Zugeben eines mit Wasser mischbaren, organischen Lösungsmittels zur wässrigen Lösung, bevor jedwede Gelierung aufzutreten beginnt, um die Ausfällung des β-Glucan-Feststoffs zu bewirken.

9. Verfahren nach Anspruch 8, worin die wässrige Lösung von β-Glucan durch Lösen von β-Glucan in Wasser bei einer Temperatur von größer als ungefähr 70 °C, bevorzugt größer als ungefähr 90 °C, am bevorzugtesten größer als ungefähr 100 °C hergestellt wird.

10. Verfahren nach Anspruch 8, worin die wässrige Lösung von β-Glucan durch Erhitzen eines wässrigen β-Glucan-Gels, optional mit zusätzlichem Wasser, bei einer Temperatur von größer als ungefähr 70 °C, bevorzugt größer als ungefähr 90 °C, am bevorzugtesten größer als ungefähr 100 °C hergestellt wird.

11. Verfahren nach einem der Ansprüche 8 bis 10, worin das Wasser aus der wässrigen Lösung durch Verdampfung entfernt wird, wobei das Wasser bevorzugt bei einer Temperatur von höher als der Schmelztemperatur eines Gels, das geeignet ist, sich durch das β-Glucan zu bilden, verdampft wird.

12. Verfahren nach einem der Ansprüche 8 bis 11, worin das mit Wasser mischbare, organische Lösungsmittel Ethanol ist.

13. Verfahren nach einem der Ansprüche 8 bis 12, worin die wässrige Lösung von β-Glucan durch Folgendes erhalten wird:
- Mahlen von Getreidekorn zur Bildung eines Mehls,
- Mischen des Mehls mit Wasser zur Bildung einer Aufschlämmung einer wässrigen Lösung von β-Glucan und eines festen Rückstands, und
- Abtrennen der wässrigen Lösung von dem festen Rückstand.

14. Verfahren nach Anspruch 13, worin die wässrige Lösung gereinigt wird, bevor die Schritte von Anspruch 8 ausgeführt werden.

15. Pharmazeutische Zusammensetzung, die β-Glucan nach einem der Ansprüche 1 bis 7 und einen pharmazeutisch verträglichen Träger enthält.

16. Verwendung von β-Glucan nach Anspruch 1 als Lebensmittelzutat, wobei die Lebensmittelzutat bevorzugt eine Zutat in einem funktionellen Lebensmittel oder einem Nutrazeutikum ist.

17. Verwendung von β-Glucan nach Anspruch 1 bei der Herstellung eines essbaren Überzugs.

18. Lebensmittel, bevorzugt ein funktionelles Lebensmittel, das β-Glucan nach Anspruch 1 enthält.

19. Nutrazeutikum, das β-Glucan nach Anspruch 1 enthält.

20. Essbarer Überzug, der β-Glucan nach Anspruch 1 enthält.

## Revendications

1. β-glucane solide qui est soluble dans l'eau à plus de 2 % en poids lorsqu'il est mélangé à de l'eau à une température inférieure à environ 50°C et ledit β-glucane solide étant capable de former un gel.

2. β-glucane selon la revendication 1 où la température est inférieure à environ 30°C.

3. β-glucane selon la revendication 1 ou la revendication 2 qui est capable de former un gel en dessous d'environ 70°C.

4. β-glucane selon l'une quelconque des revendications 1 à 3 qui est soluble dans l'eau à plus de 50 % en poids, de préférence à plus de 80 % en poids, plus préférablement à environ 100 % en poids, lorsqu'il est mélangé à de l'eau à une température inférieure à environ 50°C.

5. β-glucane selon l'une quelconque des revendications 1 à 4 qui a un poids moléculaire moyen de moins d'environ 200000 Daltons.

6. β-glucane selon l'une quelconque des revendications 1 à 5 qui est capable de former un gel mou lorsqu'il est dissous dans de l'eau à une concentration supérieure à environ 2 % en poids, ou un gel fluide lorsqu'il est dissous dans de l'eau à une concentration inférieure à environ 2 % en poids.

7. β-glucane selon l'une quelconque des revendications 1 à 6 qui a été obtenu à partir d'une céréale, la céréale étant de préférence choisie parmi l'orge, l'avoine ou le blé.

8. Procédé de préparation de β-glucane selon la revendication 1 à partir d'une solution aqueuse de β-glucane qui inclut les étapes consistant à :
- enlever l'eau de la solution aqueuse avant qu'une quelconque gélification ne commence à se produire pour donner du β-glucane solide, ou
- ajouter un solvant organique miscible dans l'eau à la solution aqueuse avant qu'une quelconque gélification ne commence à se produire pour provoquer la précipitation du β-glucane solide.

9. Procédé selon la revendication 8 dans lequel la solution aqueuse de β-glucane est préparée en dissolvant le β-glucane dans de l'eau à une température supérieure à environ 70°C, de préférence supérieure à environ 90°C, plus préférablement supérieure à environ 100°C.

10. Procédé selon la revendication 8 dans lequel la solution aqueuse de β-glucane est préparée en chauffant un gel de β-glucane aqueux, éventuellement avec de l'eau supplémentaire, à une température supérieure à environ 70°C, de préférence supérieure à environ 90°C, plus préférablement supérieure à environ 100°C.

11. Procédé selon l'une quelconque des revendications 8 à 10 dans lequel l'eau est enlevée de la solution aqueuse par évaporation, l'eau étant de préférence évaporée à une température supérieure à la température de fusion d'un gel pouvant être formé par le β-glucane.

12. Procédé selon l'une quelconque des revendications 8 à 11 dans lequel le solvant organique miscible dans l'eau est l'éthanol.

13. Procédé selon l'une quelconque des revendications 8 à 12 dans lequel la solution aqueuse de β-glucane est obtenue par :
- broyage de grains de céréales pour former une farine,
- mélangeage de la farine avec de l'eau pour former une pâte constituée d'une solution aqueuse de β-glucane et d'un résidu solide, et
- séparation de la solution aqueuse du résidu solide.

14. Procédé selon la revendication 13 dans lequel la solution aqueuse est purifiée avant d'effectuer les étapes de la revendication 8.

15. Composition pharmaceutique qui contient du β-glucane selon l'une quelconque des revendications 1 à 7 et un véhicule pharmaceutiquement acceptable.

16. Utilisation du β-glucane selon la revendication 1 en tant qu'ingrédient alimentaire, l'ingrédient alimentaire étant de préférence un ingrédient dans un aliment fonctionnel ou un nutraceutique.

17. Utilisation du β-glucane selon la revendication 1 dans la préparation d'un film comestible.

18. Aliment, de préférence aliment fonctionnel, qui contient du β-glucane selon la revendication 1.

19. Nutraceutique qui contient du β-glucane selon la revendication 1.

20. Film comestible qui contient du β-glucane selon la revendication 1.
